# EUROPÄISCHE PATENTSCHRIFT

(11) **EP 2 853 195 B1**
(45) Veröffentlichungstag und Bekanntmachung des Hinweises auf die Patenterteilung: **27.06.2018**
(21) Anmeldenummer: 14186656.6
(22) Anmeldetag: 26.09.2014
(51) Int. Cl.: A61B 5/0428, A61B 5/00, A61B 5/18, A61B 5/0408

(54) **Sitz oder Liege in einem Fahrzeug mit einem Sensor zur berührungslosen elektrokardiographischen Messung**
Seat or couch in a vehicle with a sensor for non-contact electrocardiographic measurement
Siège pour un véhicule avec un capteur pour la mesure électrocardiographique sans contact

(30) Priorität: 27.09.2013 DE 102013219513
(43) Veröffentlichungstag der Anmeldung: 01.04.2015
(73) Patentinhaber: Ford Global Technologies, LLC, Dearborn, MI 48126 (US)
(72) Erfinder: Lem, Jeroen, 6213 GE Maastricht (NL); Mathissen, Marcel, 52146 Würselen (DE); Lindner, Hans-Joachim, 53881 Euskirchen (DE); Vogt, Rainer, 52072 Aachen (DE); Eilebrecht, Benjamin, 44623 Herne (DE); Walter, Marian, 52070 Aachen (DE); Leonhardt, Steffen, 52074 Aachen (DE)
(74) Vertreter: Dörfler, Thomas

(56) Entgegenhaltungen:
- DE-U1-202012 001 096
- FR-A1- 2 979 029
- US-A- 4 476 871
- US-A1- 2012 215 076
- TOBIAS WARTZEK ET AL: "ECG on the Road: Robust and Unobtrusive Estimation of Heart Rate", IEEE TRANSACTIONS ON BIOMEDICAL ENGINEERING, IEEE SERVICE CENTER, PISCATAWAY, NJ, USA, Bd. 58, Nr. 11, 1. November 2011 (2011-11-01), Seiten 3112-3120, XP011362853, ISSN: 0018-9294, DOI: 10.1109/TBME.2011.2163715

## Beschreibung

Die vorliegende Erfindung betrifft einen Sitz oder eine Liege in einem Fahrzeug mit einem Sensor zur berührungslosen elektrokardiographischen Messung an einer auf dem Sitz oder der Liege befindlichen Person.

Die Messung des elektrischen Potentials bzw. der elektrischen Feldstärke auf der Haut einer Person mittels elektrokardiographischer Sensoren bildet die Grundlage vieler medizinischer Diagnoseverfahren. Beispielsweise kann auf diesem Wege ein Elektrokardiogramm (EKG) aufgenommen werden oder aus den gemessenen elektrischen Potentialen die Herzfrequenz ermittelt werden.

Bei herkömmlichen Messverfahren zur Messung des elektrischen Potentials auf der Haut wird dieses durch Elektroden erfasst, die in direktem elektrischen Kontakt mit der Hautoberfläche stehen. Es wird also eine elektrisch leitende Verbindung zwischen der Haut einerseits und der Elektrode andererseits hergestellt. Es erweist sich hierbei jedoch häufig als schwierig, einen hinreichend guten elektrischen Kontakt zwischen der Elektrode und der Haut und damit dem Körper der zu untersuchenden Person sicherzustellen. Außerdem wird der Einsatz derartiger Diagnoseverfahren zunehmend auch in Anwendungsgebieten vorgesehen, in denen ein direkter Zugang zur Haut der zu untersuchenden Person nicht gegeben ist, wie zum Beispiel in Fahrzeuganwendungen zur Überwachung von Körperfunktionen und/oder Vitalparametern von Fahrzeuginsassen auf Sitzen oder Liegen.

So offenbart beispielsweise die US 7 684 854 B2 einen Sensor zur berührungslosen elektrokardiographischen Messung an einer Person. Die Person kann sich hierbei in einem Stuhl, einem Bett oder einem Fahrzeugsitz befinden. Das Elektrokardiogramm kann von dem Körper der eine Kleidung tragenden Person ohne direkten Kontakt zur Haut aufgenommen werden. Der Sensor umfasst eine elektrisch leitfähige, flächige Elektrode, die eine der Person zugewandte Messfläche und eine der Person abgewandte, der Messfläche gegenüberliegende Anschlussfläche aufweist, die elektrisch mit einem Vorverstärker verbunden ist. Die Elektrode und der Vorverstärker des Sensors sind von einer Abschirmung umgeben.

Einen weiteren berührungslosen Sensor zur Aufnahme eines Elektrokardiogramms einer Person offenbart die EP 2 532 306 A1. Der Sensor umfasst eine elektrisch leitfähige Elektrode und eine Detektionseinrichtung, die elektrisch mit der Elektrode verbunden und ausgelegt ist, die von der Elektrode aufgenommenen Signale zu verstärken. Der Sensor ist dazu vorgesehen, in einem Fahrzeugsitz angeordnet zu sein und bestimmte physiologische Parameter eines in dem Fahrzeugsitz sitzenden Fahrers zu ermitteln.

Die DE 20 2012 001 096 U1 offenbart kapazitive Sensoren zur kapazitiven Erfassung von Vitalparametern eines Fahrers eines Fahrzeugs. Hierzu sind die Sensoren in oder an der Rückenlehne des Sitzes des Fahrzeugs angebracht. Insbesondere wird gemäß einer Ausführungsform vorgeschlagen, die Sensoren in oder an der Rückenlehne des Sitzes in zwei voneinander mit einem der Breite der Wirbelsäule des Fahrers entsprechenden Abstand getrennten Reihen verteilt anzuordnen. Je Reihe sind die Sensoren mit einer Fläche von 16 bis 36 cm² in gleichen Abständen von 1 bis 5 cm zueinander angeordnet. In einer weiteren Ausführungsform sind anstelle der zwei voneinander getrennten Sensorreihen mit über die gesamte Höhe des Sitzes in einem Abstand von 1-5 cm verteilten Sensoren zwei voneinander mit einem der Wirbelsäule entsprechenden Abstand getrennte Foliensensoren mit einer Breite von 4 bis 10 cm über die gesamte Sitzhöhe angeordnet.

Ferner offenbart die DE 10 2008 049 112 A1 eine kapazitive Textilelektrode zum Messen von Körperfunktionen und/oder Vitalparametern von Personen für Fahrzeuganwendungen, zum Beispiel in einem Sitz oder einer Liege, die einen mehrschichtigen Aufbau aufweist. Dieser umfasst zwei Textilschichten, die jeweils einen elektrisch leitfähigen Elektrodenbereich aufweisen, wobei eine weitere Textilschicht zum Herstellen eines Abstands zwischen den zwei anderen Textilschichten vorgesehen ist.

Des Weiteren ist aus der FR 2 979 029 A1 eine in einem Kissen angeordnete Personenüberwachungsvorrichtung bekannt. Das Kissen umfasst an seiner Oberseite und an seiner Unterseite jeweils eine elektrisch leitfähige, flexible Platte und ein zwischen den Platten angeordnetes elektrisch isolierendes, elastisch verformbares Element. Durch Ausübung eines Drucks auf das Kissen wird das elastisch verformbare Element zusammengedrückt. Dies erlaubt, dass sich die beiden Platten in diesem Zustand berühren und somit einen elektrischen Schaltkreis schließen.

Weiterhin offenbart die US 2012/0215076 A1 eine Anordnung für eine berührungslose elektrokardiographische Messung an einer Person. Die Anordnung umfasst Sensoren, die jeweils eine Elektrode aufweisen, die wiederum über einen elektrischen Schaltkontakt mit einer Messeinrichtung verbunden sind. Der Schaltkontakt verbindet die Elektrode elektrisch mit der Messeinrichtung, sobald von der Person ein ausreichend hoher Druck auf den Sensor ausgeübt wird, und trennt die Elektrode elektrisch von der Messeinrichtung, wenn die Person keinen Druck auf den Sensor ausübt.

Aus der US 4 476 871 A ist ferner ein Sensor zur Überwachung einer Gebärmutterhalserweiterung an einer Person bekannt, der mehrere elektrisch leitfähige, flächige Elektroden aufweist. Die Elektroden weisen eine der Person zugewandte Messfläche und eine der Person abgewandte Anschlussfläche auf. Gegenüberliegend der Anschlussfläche und beabstandet zu dieser ist ein elektrischen Anschlusskontakt zum Anschluss einer Messeinrichtung vorgesehen. Die Anschlussfläche ist erst mittels eines auf die Messfläche aufbringbaren, in Richtung des Anschlusskontakts wirkenden Drucks mit dem Anschlusskontakt elektrisch verbindbar.

Um ein zuverlässiges und stabiles Signal von den vorbekannten Sensoren bzw. Sensoranordnungen/Sensorarrays zur berührungslosen elektrokardiographischen Messung an Personen in Fahrzeuganwendungen zu erhalten, ist es wesentlich, dass die Sensoren am besten vollständig von dem Körper bzw. dem zu untersuchenden Körperbereich der Person bedeckt werden und ein möglichst geringer Abstand zwischen den Sensoren und der zu untersuchenden Person gewährleistet ist. Allgemein führen solche Elektroden, die einen großen Abstand zum Beispiel zum Rücken der zu untersuchenden Person haben, zu einem schlechten oder sogar unbrauchbaren Signal.

Man kann daher grundsätzlich versuchen, die Sensoren zum Beispiel an einer Sitz- oder Liegefläche dort zu platzieren, wo ein möglichst guter Kontakt zwischen der in dem Sitz oder auf der Liege befindlichen Person und dem Sitz oder der Liege zu erwarten ist. Ebenso ist es denkbar, insgesamt mehr Elektroden zu verwenden, um den Kontakt zwischen diesen und der zu untersuchenden Person zu verbessern. Jedoch wird wahrscheinlich die mit der Vergrößerung der Anzahl an Sensoren einhergehende Verkleinerung der Elektrodenfläche zu einer geringeren Empfindlichkeit der Sensoren führen. Eine weitere Möglichkeit könnte beispielsweise auch darin bestehen, größere Elektrodenflächen zu verwenden. Jedoch führt dies bei kleinen oder dünnen Personen dazu, dass ganze Elektrodenabschnitte nicht von dem zu untersuchenden Körper der Person bedeckt werden und somit keinen Kontakt zur Person haben, was wahrscheinlich wieder zu einer erhöhten Anfälligkeit für Störungen führt.

Vor diesem Hintergrund liegt der vorliegenden Erfindung die Aufgabe zugrunde, einen Sensor, ein Sensorarray sowie einen Sitz oder eine Liege zur berührungslosen elektrokardiographischen Messung an Personen, bevorzugt in Fahrzeuganwendungen, anzugeben, mit denen zuverlässige Aussagen über die Körperfunktionen und/oder Vitalparameter der Person gemacht werden können, das heißt, die jederzeit ein zuverlässiges Signal mit guter Signalqualität zu liefern imstande sind.

Diese Aufgabe wird durch einen Sitz oder eine Liege mit den Merkmalen des Anspruchs 1 gelöst. Weitere, besonders vorteilhafte Ausgestaltungen der Erfindung offenbaren die Unteransprüche.

Es ist darauf hinzuweisen, dass die in den Ansprüchen einzeln aufgeführten Merkmale in beliebiger, technisch sinnvoller Weise miteinander kombiniert werden können und weitere Ausgestaltungen der Erfindung aufzeigen. Die Beschreibung charakterisiert und spezifiziert die Erfindung insbesondere im Zusammenhang mit den Figuren zusätzlich.

Erfindungsgemäß weist ein Sitz oder eine Liege in einem Fahrzeug wenigstens einen Sensor zur berührungslosen elektrokardiographischen Messung an einer auf dem Sitz oder der Liege befindlichen Person auf, wobei der Sensor wenigstens eine elektrisch leitfähige, flächige Elektrode aufweist, die eine der Person zugewandte Messfläche und eine der Person abgewandte, der Messfläche gegenüberliegende Anschlussfläche umfasst. "Berührungslos" ist im Sinne der vorliegenden Erfindung derart aufzufassen, dass die Messfläche der Elektrode die Haut der zu untersuchenden Person nicht unmittelbar berührt. Beispielsweise können Kleidungsstücke zwischen der zu untersuchenden Person und der Messfläche der Elektrode angeordnet sein.

Gegenüberliegend der Anschlussfläche und beabstandet zu dieser ist gemäß der vorliegenden Erfindung ein elektrischer Anschlusskontakt zum Anschluss einer Messeinrichtung angeordnet. Als Messeinrichtung ist im Sinne der vorliegenden Erfindung jedwede Einrichtung, zum Beispiel in Form einer elektronischen Schaltung, zu verstehen, die geeignet ist, ein von der Elektrode aufgenommenes Messsignal in irgendeiner Form aufzubereiten, zum Beispiel zu verstärken.

Erfindungsgemäß ist die Anschlussfläche erst mittels eines auf die Messfläche der Elektrode aufbringbaren, in Richtung des Anschlusskontakts wirkenden Drucks mit dem Anschlusskontakt elektrisch verbindbar. Das heißt, solange kein zur Herstellung der elektrischen Verbindung ausreichender Druck auf die Messfläche der Elektrode in Richtung des Anschlusskontakts einwirkt, ist die Elektrode elektrisch nicht mit dem Anschlusskontakt verbunden und wird somit kein Signal von der Elektrode zur Messeinrichtung übertragen. Somit ist stets sichergestellt, dass der Sensor erst dann automatisch ein Signal an die Messeinrichtung liefert, wenn ein ausreichender Kontakt zwischen dem Sensor und der zu untersuchenden Person besteht, indem diese mit einem der Lage des Sensors entsprechenden Körperteil gegen den Sensor drückt. Besteht dieser Kontakt zwischen dem Sensor und der zu untersuchenden Person nicht, das heißt, ist das von der Messfläche aufgenommene Signal mit hoher Wahrscheinlichkeit ein fehlerhaftes und damit unbrauchbares Signal, liefert der erfindungsgemäße Sensor der Messeinrichtung dieses Signal nicht. Folglich können zuverlässige Aussagen über die Körperfunktionen und/oder Vitalparameter der Person gemacht werden, da der Sensor zu jeder Zeit ein zuverlässiges Signal mit guter Signalqualität liefert, nämlich entweder das brauchbare Messsignal der Elektrode oder kein Signal.

Gemäß der Erfindung ist ein elektrischer Schaltkontakt zwischen der Anschlussfläche und dem Anschlusskontakt angeordnet und stellt die elektrische Verbindung zwischen diesen her. Der elektrische Schaltkontakt ist hierbei derart ausgelegt, die elektrische Verbindung infolge der von dem Druck auf die Messfläche der Elektrode verursachten Annäherung der Anschlussfläche der Elektrode an den Anschlusskontakt herzustellen. Hierzu kann im Prinzip jeder herkömmliche Schaltkontakt verwendet werden, welcher den elektrischen Kontakt zwischen der Anschlussfläche der Elektrode und dem Anschlusskontakt bei Annäherung der Anschlussfläche an den Anschlusskontakt schließen kann.

Eine erste vorteilhafte, in ihrer Herstellung besonders einfache Ausgestaltung der Erfindung sieht vor, dass der Schaltkontakt einen starren Kontaktstift umfasst, dessen Länge sich in eine Richtung von der Anschlussfläche der Elektrode zum Anschlusskontakt erstreckt. Der Kontaktstift kann hierbei entweder an der Anschlussfläche der Elektrode oder an dem Anschlusskontakt befestigt sein. Auf diese Weise wird abhängig von der Länge des Kontaktstifts und dem drucklosen Abstand zwischen der Anschlussfläche und dem Anschlusskontakt die elektrische Verbindung zwischen der Anschlussfläche der Elektrode und dem Anschlusskontakt ab einem vorherbestimmbaren Druck auf die Messfläche der Elektrode hergestellt.

Eine alternative vorteilhafte Ausgestaltung der Erfindung sieht vor, dass der Schaltkontakt einen Kontaktstift umfasst, dessen Länge sich in eine Richtung von der Anschlussfläche der Elektrode zum Anschlusskontakt erstreckt, wobei die Länge gegen eine am Kontaktstift angreifende Federkraft verkürzbar ist. Beispielsweise könnte der Kontaktstift aus wenigstens zwei ineinander angeordneten Kontakthülsen ausgebildet sein, die gegen die Federkraft relativ zueinander verschiebbar bzw. teleskopierbar sind. Mit anderen Worten lässt sich der Kontaktstift des Schaltkontakts auch nach dem Herstellen der elektrischen Verbindung zwischen der Anschlussfläche der Elektrode und dem Anschlusskontakt gegen die an dem Kontaktstift angreifenden Federkraft zusammenschieben. Somit wird entsprechend dem auf die Messfläche der Elektrode aufgebrachten Druck eine weitere Annäherung der Elektrode an den Anschlusskontakt ermöglicht, auch wenn die elektrische Verbindung bereits hergestellt ist.

Gemäß der Erfindung sind mehrere Elektroden pro Sensor vorgesehen. Die einzelnen Elektroden des Sensors können hierbei eine sich aus der Gesamtheit der einzelnen kleineren Elektroden definierende, zusammengesetzte größere Gesamtelektrodenfläche bilden, die durch die einzelnen Elektroden segmentiert ist. Der wesentliche Vorteil dieser Ausgestaltung ist, dass auf diese Weise lediglich diejenigen Elektrodenbereiche der Gesamtelektrodenfläche automatisch ein Messsignal liefern, auf die durch die zu untersuchende Person ein ausreichend hoher Druck auf die Messfläche der jeweiligen Elektrode ausgeübt wird. Andere Elektrodenbereiche liefern hingegen kein Messsignal.

Eine vorteilhafte Ausgestaltung der Erfindung sieht vor, dass die mehreren Elektroden jeweils eine Messfläche von etwa 1 cm² bis 16 cm², vorzugsweise etwa 4 cm², aufweisen und in einem Abstand von weniger als etwa 1 cm, vorzugsweise weniger als etwa 5 mm, zueinander matrixartig angeordnet sind. Dementsprechend lässt sich die aus der Gesamtheit der einzelnen Elektroden definierende Gesamtelektrodenfläche in relativ kleine Elektrodenbereiche segmentieren, die bei einem ausreichenden Druck auf die jeweiligen Messflächen der Elektroden automatisch ein zuverlässiges Messsignal liefern.

Neben dieser matrixartigen Anordnung können die Sensoren auch in beliebig anderen Muster angeordnet sein, z. B. spiralförmig, kreisförmig oder stochastisch.

Gemäß einer weiteren vorteilhaften Ausgestaltung der Erfindung ist eine kompressible Zwischenmaterialschicht zwischen die Anschlussfläche der Elektrode und dem Anschlusskontakt eingefügt. Somit lässt sich anhand der kompressiblen Zwischenmaterialschicht der gewünschte Druck genau festlegen, der zum Herstellen der elektrischen Verbindung zwischen der Anschlussfläche der Elektrode und dem Anschlusskontakt erforderlich ist. Ferner stellt die Zwischenmaterialschicht einen genau definierten Abstand im drucklosen Zustand zwischen der Anschlussfläche der Elektrode und dem Anschlusskontakt sicher.

Ein Sensorarray kann wenigstens zwei Sensoren der vorbeschriebenen erfindungsgemäßen Art umfassen. Als Sensorarray ist im Sinne der vorliegenden Erfindung jede Art von Anordnung mehrerer dieser Sensoren zu verstehen.

Hierbei können diese Sensoren in zwei zueinander beabstandeten Reihen angeordnet sein, wobei je Reihe jeweils mehrere Sensoren mit jeweils einer Messfläche von etwa 1 cm² bis 16 cm², vorzugsweise etwa 4 cm², in einem Abstand von weniger als etwa 1 cm, insbesondere weniger als etwa 5 mm, zueinander matrixartig angeordnet sind. Dementsprechend lässt sich eine dichte Anordnung relativ kleiner Sensoren für das Sensorarray realisieren, die in ihrer Gesamtheit eine größere Fläche abdecken können, wobei im Anwendungsfall lediglich diejenigen Sensoren automatisch an die jeweilige Messeinrichtung ein Messsignal liefern, die auf ihrer Messfläche mit einem ausreichend großen Druck beaufschlagt sind und somit einen ausreichend guten Kontakt zu der mit dem Sensorarray zu untersuchenden Person aufweisen.

Gemäß der vorliegenden Erfindung weist ein Sitz oder eine Liege in einem Fahrzeug wenigstens einen Sensor oder wenigstens ein Sensorarray nach der vorbeschriebenen, erfindungsgemäßen Art zur berührungslosen elektrokardiographischen Messung an einer auf dem Sitz oder der Liege befindlichen Person auf.

Weitere Merkmale und Vorteile der Erfindung ergeben sich aus der folgenden Beschreibung nicht einschränkend zu verstehender Ausführungsbeispiele der Erfindung, die im Folgenden unter Bezugnahme auf die Zeichnung näher erläutert werden. In dieser Zeichnung zeigen schematisch:
- Fig. 1: ein Sensorarray und einen Sitz für ein Fahrzeug nach dem Stand der Technik,
- Fig. 2: eine Querschnittsansicht eines erfindungsgemäßen Sensors entsprechend einer ersten Ausführungsform,
- Fig. 3: eine Querschnittsansicht eines erfindungsgemäßen Sensors entsprechend einer weiteren Ausführungsform und
- Fig. 4: ein Sensorarray und einen Sitz für ein Fahrzeug gemäß einer erfindungsgemäßen Ausführungsform.

In den unterschiedlichen Figuren sind gleiche Teile stets mit denselben Bezugszeichen versehen, so dass diese in der Regel auch nur einmal beschrieben werden.

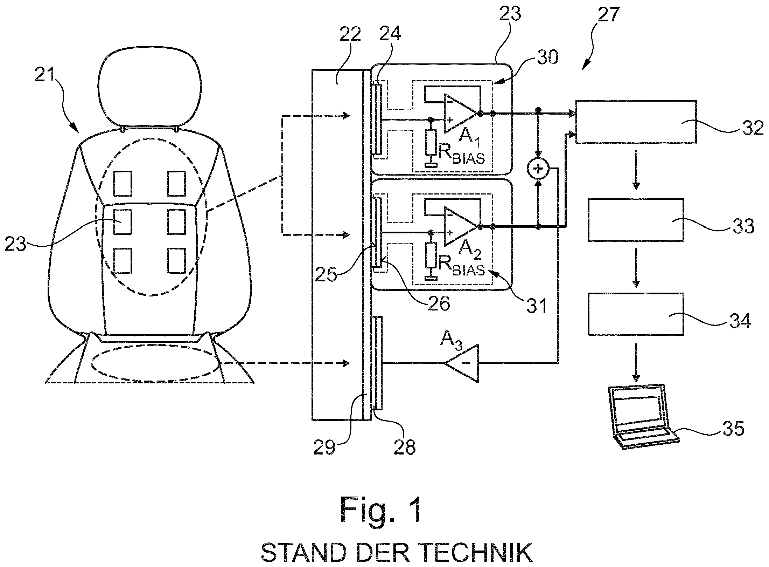

Fig. 1 stellt schematisch ein Sensorarray 20 und einen Sitz 21 für ein Fahrzeug zur berührungslosen elektrokardiographischen Messung an einer Person 22 nach dem Stand der Technik dar. Wie zu erkennen ist, besteht das Sensorarray aus einer matrixartigen Anordnung von sechs in einer Rückenlehne eines Fahrzeugsitzes in einer 3x2-Matrix angeordneten Sensoren 23, die jeweils eine elektrisch leitfähige, flächige Elektrode 24 aufweisen. In der Sitzfläche des Fahrzeugsitzes 21 ist ferner eine weitere Elektrode angeordnet, über die ein Bezugspotential der Schaltung eingeprägt wird.

Jede Elektrode 24 umfasst eine der Person 22 bzw. ihrem Körper zugewandte Messfläche 25 und eine der Person abgewandte, der Messfläche 25 gegenüberliegende Anschlussfläche 26 zum Anschluss einer Messeinrichtung 27. Wie in Fig. 1 dargestellt ist, berührt die Messfläche 25 der einzelnen Elektroden 24 die Haut der zu untersuchenden Person 22 nicht unmittelbar. Vielmehr ist auf der Messfläche 25 jeder Elektrode 24 in Fig. 1 eine Isolierung 28 aufgebracht. Außerdem befindet sich zwischen dem Körper der zu untersuchenden Person 22 und der Messfläche 25 ferner noch die von der Person getragene Kleidung 29.

Die in Fig. 1 dargestellte Messeinrichtung 27 umfasst pro Sensor 23 einen von einer Abschirmung 30 umgebenen Vorverstärker 31. Ferner verstärkt ein Instrumentenverstärker 32 das von den Elektroden 24 der Sensoren 23 aufgenommene Messsignal, gefolgt von einer Filter- und Verstärkungseinheit 33 sowie einem A/D-Wandler 34. Das von dem A/D-Wandler 34 ausgegebene digitale Messsignal kann anschließend beispielsweise mittels einer digitalen Rechnereinheit 35 in geeigneter Weise weiterverarbeitet werden.

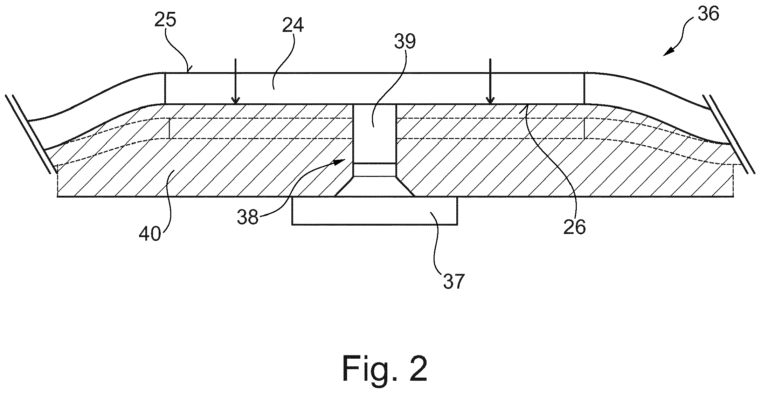

Fig. 2 stellt schematisch eine Querschnittsansicht eines erfindungsgemäßen Sensors 36 zur berührungslosen elektrokardiographischen Messung an einer Person entsprechend einer ersten Ausführungsform dar. Der in Fig. 2 dargestellte Sensor 36 umfasst eine elektrisch leitfähige, flächige Elektrode 24, die eine der Person zugewandte Messfläche 25 und eine der Person abgewandte, der Messfläche 25 gegenüberliegende Anschlussfläche 26 umfasst. Gegenüberliegend der Anschlussfläche 26 und beabstandet zu dieser ist ein elektrischer Anschlusskontakt 37 zum Anschluss einer in Fig. 2 nicht dargestellten Messeinrichtung angeordnet. Wie in Fig. 2 durch die gestrichelte Darstellung angedeutet ist, ist die Anschlussfläche 26 der Elektrode 24 mittels eines auf die Messfläche 25 aufbringbaren Drucks in Richtung des Anschlusskontakts 37 bewegbar. Hierbei ist die Anschlussfläche 26 über einen zwischen der Anschlussfläche 26 und dem Anschlusskontakt 37 angeordneten Schaltkontakt 38 mit dem Anschlusskontakt 37 elektrisch verbindbar.

Bei dem in Fig. 2 dargestellten Ausführungsbeispiel ist der Schaltkontakt 38 in Form eines im Wesentlichen starren Kontaktstifts 39 ausgebildet, dessen Länge sich in die Richtung von der Anschlussfläche 26 der Elektrode 24 zum Anschlusskontakt 37 erstreckt. Sobald ein ausreichend hoher Druck auf die Messfläche 25 der Elektrode 24 in Richtung des Anschlusskontakts 37 einwirkt, stellt der Kontaktstift 39 die elektrische Verbindung zwischen der Anschlussfläche 26 und dem Anschlusskontakt 37 her. Ein von der Elektrode 24 aufgenommenes Messsignal kann dann an eine mit dem Anschlusskontakt 37 elektrisch verbundene Messeinrichtung (nicht dargestellt) weitergeleitet werden. Bei einem unzureichenden Druck auf die Messfläche 25 der Elektrode 24 öffnet der Schaltkontakt 38, so dass keine Messsignale mehr an die Messeinrichtung weitergeleitet werden.

Um den zum Schließen des Schaltkontakts 38 erforderlichen Druck genau definieren und vorgeben zu können sowie den Abstand zwischen der Anschlussfläche 26 und dem Anschlusskontakt 37, ist bei dem in Fig. 2 dargestellten Ausführungsbeispiel des Sensors 36 eine entsprechend kompressible Zwischenmaterialschicht 40 zwischen die Anschlussfläche 26 der Elektrode 24 und dem Anschlusskontakt 37 eingefügt. Im Bereich des Schaltkontakts 38 weist die Zwischenmaterialschicht 40 eine entsprechende Ausnehmung für den Schaltkontakt 38 auf.

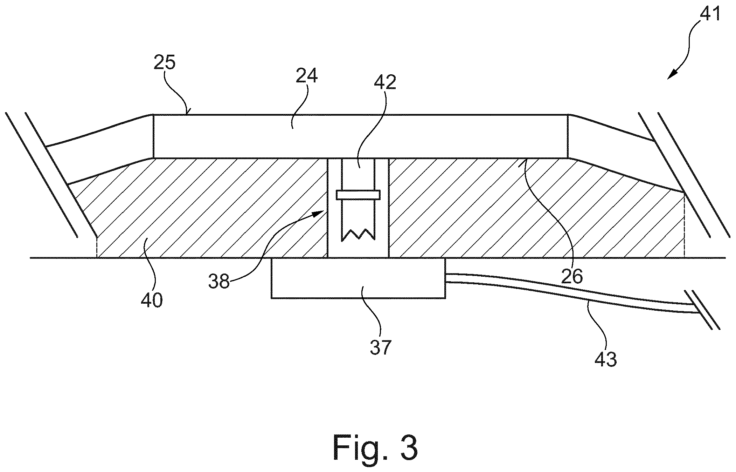

In Fig. 3 ist schematisch eine Querschnittsansicht eines erfindungsgemäßen Sensors 41 entsprechend einer weiteren Ausführungsform dargestellt. Der wesentliche Unterschied zur in Fig. 2 gezeigten Ausführungsform besteht in der Ausbildung des Schaltkontakts 38. Bei der in Fig. 3 dargestellten Ausführungsform umfasst der Schaltkontakt 38 einen Kontaktstift 42, dessen Länge sich in eine Richtung von der Anschlussfläche 26 der Elektrode 24 zum Anschlusskontakt 37 erstreckt, wobei die Länge gegen eine am Kontaktstift 42 angreifende Federkraft verkürzbar ist. Zum Beispiel kann der Kontaktstift aus zwei ineinander angeordneten Kontakthülsen ausgebildet sein, die gegen die Federkraft relativ zueinander verschiebbar bzw. teleskopierbar sind. Mit anderen Worten lässt sich der Kontaktstift 42 des Schaltkontakts 38 auch nach dem Herstellen der elektrischen Verbindung zwischen der Anschlussfläche 26 der Elektrode 24 und dem Anschlusskontakt 37 gegen die an dem Kontaktstift 42 angreifende Federkraft zusammenschieben. Somit wird entsprechend dem auf die Messfläche 25 der Elektrode 24 aufgebrachten Druck eine weitere Annäherung der Elektrode 24 an den Anschlusskontakt 37 ermöglicht, auch wenn die elektrische Verbindung bereits hergestellt ist.

Ferner ist in Fig. 3 ein Kabel 43 zum Anschluss einer nicht dargestellten Messeinrichtung an den Anschlusskontakt 37 erkennbar.

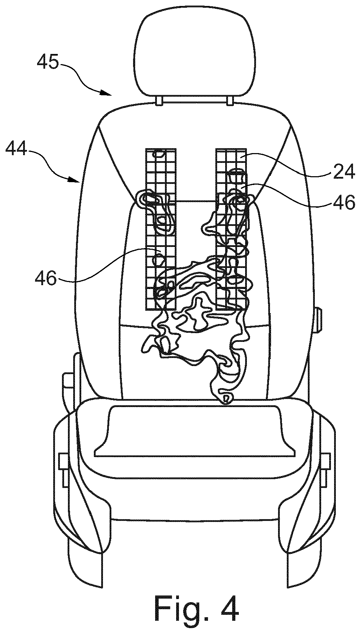

In Fig. 4 sind ein Sensorarray 44 und ein Sitz 45 für ein Fahrzeug zur berührungslosen elektrokardiographischen Messung an einer Person gemäß einer erfindungsgemäßen Ausführungsform dargestellt. Das Sensorarray 44 ist in der Rückenlehne des Fahrzeugsitzes 45 angeordnet und umfasst bei dem gezeigten Ausführungsbeispiel zwei in einem Abstand zueinander angeordnete, längliche Sensoren 46, die jeweils aus mehreren, in einer 3x15-Matrix angeordneten Elektroden 24 gebildet sind. Die einzelnen Elektroden 24 weisen jeweils eine Messfläche von etwa 4 cm² auf und sind in einem Abstand von weniger als etwa 5 mm zueinander matrixartig angeordnet. In Fig. 4 ist dargestellt, dass abhängig von dem augenblicklich auf die Elektroden aufgebrachten Druck durch eine in dem Fahrzeugsitz sitzenden Person lediglich einige der mehreren Elektroden 24 aktiv sind, das heißt elektrisch mit den jeweiligen Messeinrichtungen (nicht dargestellt) verbunden sind, wohingegen die restlichen Elektroden 24 des jeweiligen Sensors 46 inaktiv sind und dementsprechend elektrisch nicht mit den zugehörigen Messeinrichtungen verbunden sind. Folglich sind automatisch stets diejenigen Elektroden 24 aktiv, auf die ein ausreichend hoher Druck zum Herstellen der elektrischen Verbindung zwischen der Anschlussfläche der Elektrode 24 und dem Anschlusskontakt ausgeübt wird. Diese Elektroden 24 liefern der zugehörigen Messeinrichtung aufgrund des guten Kontakts zu der zu untersuchenden Person ein verwertbares Messsignal, wohingegen die restlichen, mit einem zu geringen oder gar keinem Druck beaufschlagten Elektroden 24 kein Messsignal an die Messeinrichtung liefern.

Der erfindungsgemäße Sensor, das Sensorarray und der Sitz oder die Liege wurden anhand mehrerer in der Figur dargestellten Ausführungsbeispiele näher erläutert. Der Sensor, das Sensorarray und der Sitz oder die Liege sind jedoch nicht auf die hierin beschriebenen Ausführungsformen beschränkt, sondern umfassen auch gleich wirkende weitere Ausführungsformen.

In bevorzugter Ausführung werden der erfindungsgemäße Sensor, das Sensorarray und der Sitz oder die Liege in einem Fahrzeug, insbesondere einem Kraftfahrzeug, zur berührungslosen elektrokardiographischen Messung an einer Person verwendet.

### Bezugszeichenliste

- 20: Sensorarray
- 21: Sitz
- 22: Person, Körper
- 23: Sensor
- 24: Elektrode
- 25: Messfläche
- 26: Anschlussfläche
- 27: Messeinrichtung
- 28: Isolierung
- 29: Kleidung
- 30: Abschirmung
- 31: Vorverstärker
- 32: Instrumentenverstärker
- 33: Filter- und Verstärkungseinheit
- 34: A/D-Wandler
- 35: Rechnereinheit
- 36: Sensor
- 37: Anschlusskontakt
- 38: Schaltkontakt
- 39: Kontaktstift
- 40: Zwischenmaterialschicht
- 41: Sensor
- 42: Kontaktstift
- 43: Kabel
- 44: Sensorarray
- 45: Sitz
- 46: Sensor

## Patentansprüche

1. Sitz oder Liege in einem Fahrzeug mit wenigstens einem Sensor (36, 41, 46) zur berührungslosen elektrokardiographischen Messung an einer auf dem Sitz (21, 45) oder der Liege befindlichen Person, wobei der Sensor (46) mehrere elektrisch leitfähige, flächige Elektroden (24) aufweist, die eine segmentierte Gesamtelektrodenfläche bilden und jeweils eine der Person zugewandte Messfläche (25) und eine der Person abgewandte, der Messfläche (25) gegenüberliegende Anschlussfläche (26) umfassen, wobei gegenüberliegend der Anschlussfläche (26) und beabstandet zu dieser ein elektrischer Anschlusskontakt (37) zum Anschluss einer Messeinrichtung angeordnet ist, wobei die Anschlussfläche (26) erst mittels eines auf die Messfläche (25) aufbringbaren, in Richtung des Anschlusskontakts (37) wirkenden Drucks mit dem Anschlusskontakt (37) elektrisch verbindbar ist und ein elektrischer Schaltkontakt (38) zwischen der Anschlussfläche (26) und dem Anschlusskontakt (37) angeordnet ist und die elektrische Verbindung zwischen diesen herstellt, wobei der Schaltkontakt (38) einen starren Kontaktstift (39) umfasst, dessen Länge sich in eine Richtung von der Anschlussfläche (26) der Elektrode (24) zum Anschlusskontakt (37) erstreckt, oder der Schaltkontakt (38) einen Kontaktstift (42) umfasst, dessen Länge sich in eine Richtung von der Anschlussfläche (26) der Elektrode (24) zum Anschlusskontakt (37) erstreckt, wobei die Länge gegen eine am Kontaktstift (42) angreifende Federkraft verkürzbar ist.

2. Sitz oder Liege nach Anspruch 1,
**dadurch gekennzeichnet, dass**
die mehreren Elektroden (24) jeweils eine Messfläche von etwa 1 cm² bis 16 cm², vorzugsweise etwa 4 cm², aufweisen und in einem Abstand von weniger als etwa 1 cm, vorzugsweise weniger als etwa 5 mm, zueinander matrixartig angeordnet sind.

3. Sitz oder Liege nach einem der vorhergehenden Ansprüche,
**dadurch gekennzeichnet, dass**
eine kompressible Zwischenmaterialschicht (39) zwischen die Anschlussfläche (26) der Elektrode (24) und dem Anschlusskontakt (37) eingefügt ist.

4. Sitz oder Liege nach einem der vorhergehenden Ansprüche,
**gekennzeichnet durch**
wenigstens zwei Sensoren (46).

5. Sitz oder Liege nach dem vorhergehenden Anspruch,
**dadurch gekennzeichnet, dass**
die Sensoren (46) länglich ausgebildet und in einem Abstand zueinander angeordnet sind, wobei jeder Sensor (46) jeweils mehrere Elektroden (24) mit jeweils einer Messfläche von etwa 1 cm² bis 16 cm², vorzugsweise etwa 4 cm², aufweist, die in einem Abstand von weniger als etwa 1 cm, vorzugsweise weniger als etwa 5 mm, zueinander matrixartig angeordnet sind.

## Claims

1. Seat or couch in a vehicle, having at least one sensor (36, 41, 46) for non-contact electrocardiographic measurement on a person on the seat (21, 45) or the couch, wherein the sensor (46) has a plurality of electrically conductive, flat electrodes (24), which form a segmented overall electrode surface and each comprise a measuring surface (25) facing the person and a connecting surface (26) facing away from the person and opposite the measuring surface (25), wherein an electrical connecting contact (37) for the connection of a measuring device is arranged opposite the connecting surface (26) and at a distance from the latter, wherein the connecting surface (26) can be connected electrically to the connecting contact (37) only by means of a pressure that can be applied to the measuring surface (25) and acts in the direction of the connecting contact (37), and an electric switching contact (38) is arranged between the connecting surface (26) and the connecting contact (37) and produces the electrical connection between the latter, wherein the switching contact (38) comprises a rigid contact pin (39), the length of which extends in a direction from the connecting surface (26) of the electrode (24) to the connecting contact (37), or the switching contact (38) comprises a contact pin (42), the length of which extends in a direction from the connecting surface (26) of the electrode (24) to the connecting contact (37), wherein the length can be shortened counter to a spring force acting on the contact pin (42).

2. Seat or couch according to Claim 1,
**characterized in that**
the plurality of electrodes (24) each have a measuring surface of about 1 cm² to 16 cm², preferably about 4 cm², and are arranged in the manner of a matrix at a distance of less than about 1 cm, preferably less than about 5 mm, from one another.

3. Seat or couch according to one of the preceding claims,
**characterized in that**
a compressible intermediate material layer (39) is inserted between the connecting surface (26) of the electrode (24) and the connecting contact (37).

4. Seat or couch according to one of the preceding claims,
**characterized by**
at least two sensors (46).

5. Seat or couch according to the preceding claim,
**characterized in that**
the sensors (46) are of elongated form and are arranged at a distance from one another, wherein each sensor (46) has in each case a plurality of electrodes (24) each having a measuring surface of about 1 cm² to 16 cm², preferably about 4 cm², which are arranged in the manner of a matrix at a distance of less than about 1 cm, preferably less than about 5 mm, from one another.

## Revendications

1. Siège ou couchette dans un véhicule comportant au moins un capteur (36, 41, 46) pour la mesure électrocardiographique sans contact sur une personne située sur le siège (21, 45) ou la couchette, dans lequel le capteur (46) comporte de multiples électrodes plates électriquement conductrices (24), qui forment une surface d'électrode totale segmentée et comprennent chacune une surface de mesure (25) tournée vers la personne et une surface de connexion (26) tournée à l'opposé de la personne et en face de la surface de mesure (25), dans lequel un contact de connexion électrique (37) destiné à la connexion d'un dispositif de mesure est disposé en face de la surface de connexion (26) et est espacé de celle-ci,
dans lequel la surface de connexion (26) ne peut être connectée électriquement au contact de connexion (37) qu'au moyen d'une pression qui peut être appliquée à la surface de mesure (25) et agit dans la direction du contact de connexion (37), et un contact de commutation électrique (38) est disposé entre la surface de connexion (26) et le contact de connexion (37) et établit la liaison électrique entre ceux-ci,
dans lequel le contact de commutation (38) comprend une broche de contact rigide (39) dont la longueur s'étend dans une direction allant de la surface de connexion (26) de l'électrode (24) au contact de connexion (37), ou le contact de commutation (38) comprend une broche de contact (42) dont la longueur s'étend dans une direction allant de la surface de connexion (26) de l'électrode (24) au contact de connexion (37), dans lequel la longueur peut être réduite en s'opposant à une force de ressort agissant sur la broche de contact (42).

2. Siège ou couchette selon la revendication 1,
**caractérisé en ce que** les multiples électrodes (24) présentent respectivement une surface de mesure d'environ 1 cm² à 16 cm², de préférence d'environ 4 cm², et sont disposées les unes par rapport aux autres en forme de matrice à une distance inférieure à environ 1 cm, de préférence inférieure à environ 5 mm.

3. Siège ou couchette selon l'une des revendications précédentes,
**caractérisé en ce qu'**une couche compressible de matériau intermédiaire (39) est insérée entre la surface de connexion (26) de l'électrode (24) et le contact de connexion (37).

4. Siège ou couchette selon l'une quelconque des revendications précédentes,
**caractérisé par** au moins deux capteurs (46).

5. Siège ou couchette selon la revendication précédente,
**caractérisé en ce que** les capteurs (46) sont réalisés de manière allongée et sont disposés de manière espacée les uns des autres, dans lequel chaque capteur (46) comporte plusieurs électrodes (24) présentant respectivement une surface de mesure d'environ 1 cm² à 16 cm², de préférence d'environ 4 cm², qui sont disposées les unes par rapport aux autres de manière matricielle à une distance inférieure à environ 1 cm, de préférence inférieure à environ 5 mm.
